# EUROPEAN PATENT APPLICATION

(11) **EP 2 666 505 A1**
(43) Date of publication of application: **27.11.2013**
(21) Application number: 13384001.7
(22) Date of filing: 22.05.2013
(51) Int. Cl.: A61M 21/00

(54) **Multisensory stimulation device**

(30) Priority: 23.05.2012 ES 201200570
(71) Applicant: Pirla Llorens, Francesc Xavier, 08225 Terrassa (ES); Armengol Giraut, Joan, La Massana (AD)
(72) Inventor: Pirla Llorens, Francesc Xavier, 08225 Terrassa (ES); Armengol Giraut, Joan, La Massana (AD)
(74) Representative: Isern-Jara, Jaime

(57) **Abstract**

Multisensory stimulation device comprises a water mattress (2) bed (7), a cover (3) for the user's head (4), equipped with an input/output audiovisual system and an input data keyboard, a computer application provided with a library of eligible emotional inducements, prepared for the selection of one of such inducements, consisting each emotional inducement of a group of resounding, chromatic and/or image outputs, sent out by the input/output audiovisual system placed in said cover (3). The computer application comprises the steps of select (110) the language of the application; select (120) the functions from a group formed by: emotional inducements (121), mental trips (122), customized inducements (123) and download of new inducements (124); select (180) the kind of the voice of the interaction and the complements like, for example, a male or female voice, rolling of the sea, aroma or ionization; and implement (190) the selected function.

## Description

### TECHNICAL FIELD

The present invention relates to a multisensory stimulation device.

### BACKGROUND ART

In the neurology, psychology and psychiatry sectors, the emotional inducement is known as a particular group of sensory stimuli (colour, sound, aroma, etc.) that causes a specific emotional stimulation (relaxation, activation, concentration and the like). Currently there are known devices cooperating in obtaining some of these stimuli specifically, although in a single way.

The present invention has the purpose of providing a machine having influence on all of these stimuli or emotions according to a selection made by the user or the therapist, based on neurolinguistic programming techniques (PNL), hypnotic language, sleep therapy, acoustic and visual binaural pulses, frequency stimulators of cerebral waves, chromatic therapy and aromatherapy.

### DISCLOSURE OF THE INVENTION

Come to this result, the object of the present invention is a multisensory stimulation device, which in its essence is characterised by comprising a water mattress bed, a cover for the user's head, and it is also equipped with an input/output audiovisual system, an input data keyboard, and a computer application provided with a library of eligible emotional inducements, consisting each emotional inducement of a group of resounding, chromatic and/or image outputs, sent out by the input/output audiovisual system placed in the cover of the user's head.

Preferentially, the device comprises a touch screen.

The water mattress can be provided with a vibration system and/or a heating system.

As complements, the cover can be provided with an aroma diffuser and/or an ionizer for the inside air of the cover.

The aforementioned computer application preferably comprises the steps of, by means of the interaction of the user or the therapist with the input/output audiovisual system and the keyboard:
- Select the language of the application.
- Select the functions from a group formed by: emotional inducements, mental trips, customized inducements and download of new inducements.
- Select the kind of the voice of the interaction and the complements like, for example, a male or female voice, rolling of the sea, aroma or ionization; and
- Implement the selected function by the device.

### BRIEF DESCRIPTION OF THE DRAWINGS

Following, there is a detailed description of a preferred embodiment, but not restrictive, of the multisensory stimulation device, object of the invention. For its better understanding, there are some drawings attached for an illustrative and not restrictive example. In these drawings:
Figure 1 shows a side view of an embodiment of the multisensory stimulation device of the present invention.
Figure 2 shows a flow chart representing the flow of the programme or the algorithm of the computer application for the selection of the emotional inducement to implement.

### DETAILED DESCRIPTION OF AN EMBODIMENT OF THE INVENTION

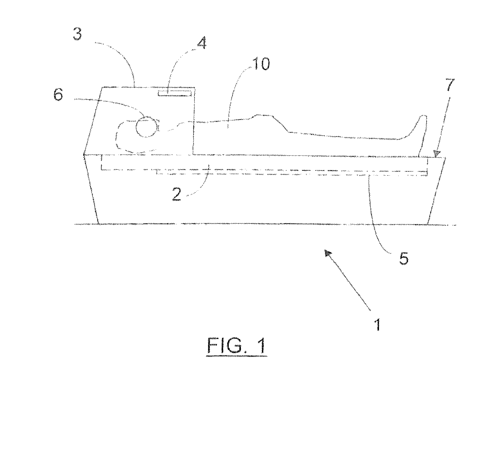

Figure 1 of these drawings shows the multisensory stimulation device (1) essentially comprising a bed (7) (it can be like the hospital driver bed or stretcher, for example) with a water mattress (2) and a cover (3) like a cubicle for the head of the user (4). The cover is equipped with an input/output audiovisual system and an input data keyboard, a computer application provided with a library of eligible emotional inducements, prepared for the selection of an emotional inducement. An emotional inducement is a group of resounding, chromatic and/or image outputs, sent out by the input/output audiovisual system, comprising a speaker (6), placed in the cover (3), inducing a particular emotional condition for a user.

The multisensory stimulation device (1) comprises a touch screen (4), an aroma diffuser and/or an ionizer placed inside the cover (3).

The water mattress (2) is provided with a vibration system and a heating system (5).

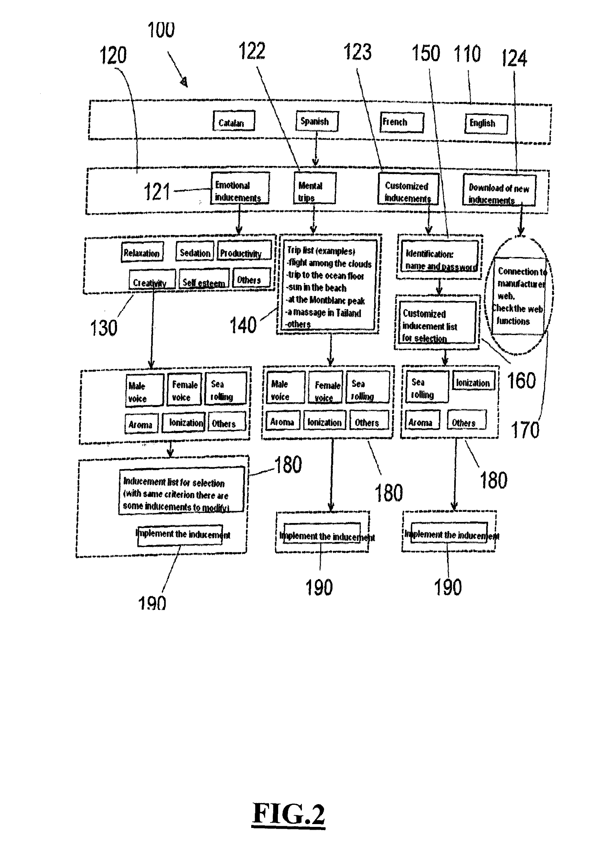

Next, the operational mode of the device of the present invention is described, of which implementation is managed and controlled by a computer application by means of the interaction of the user or the therapist with the input/output audiovisual system and the keyboard or the touch screen (4). The main algorithm (100) is showed on figure 2, comprising the steps of:
- Select (110) the language of the application, among, for example, Catalan, Spanish, French or English;
- Select (120) the functions from a group formed by: emotional inducements (121), mental trips (122), customized inducements (123) and download of new inducements (124).
- Select (180) the kind of the voice of the interaction and the complements like, for example, a male or female voice, rolling of the sea, aroma or ionization; and
- Implement (190) the selected function.

If the emotional inducements (121) are selected, they can be chosen, in the block 130, from relaxation, sedation, productivity, creativity, self esteem and the like.

Some examples of mental trips (122) can be: flights among the clouds, trips to the ocean floor, sun in the beach, on the peak of a high mountain, a massage in an exotic country, etc, eligible in the block 140.

If customized inducements are selected (123), they have been previously input in an inducements data base, and there is a user password required (150), and then the particular inducement is selected (160) from the inducements data base.

If the download of new inducement (124) is selected, a routine of selection is implemented in the block 170 by the functions manufacturer web or the available additional inducements.

As the essence of the present invention is described enough, as well as its practical execution mode, it is stated that if its fundamental principle is not modified, it can be subjected to detail variations.

## Claims

1. Multisensory stimulation device, **characterised in that** comprises a bed (7) with a water mattress (2), a cover (3) for the head of the user (4), equipped with an input/output audiovisual system and an input data keyboard, a computer application provided with a library of eligible emotional inducements, prepared for the selection of one of such inducements, consisting each emotional inducement of a group of resounding, chromatic and/or image outputs, sent out by the input/output audiovisual system placed in said cover (3).

2. Multisensory stimulation device, according to claim 1, **characterised in that** comprises a touch screen (4), placed inside the cover (3).

3. Multisensory stimulation device, according to claim 1, **characterised in that** the water mattress (2) is provided with a vibration system.

4. Multisensory stimulation device, according to claim 1, **characterised in that** the water mattress (2) is provided with a heating system (5).

5. Multisensory stimulation device, according to claim 1, **characterised in that** comprises an aroma diffuser inside the cover (3).

6. Multisensory stimulation device, according to claim 1, **characterised in that** comprises an ionizer for the inside air of the cover (3).

7. Multisensory stimulation device, according to claim 1, **characterised in that** said computer application comprises the steps of, by means of the interaction of the user or the therapist with the input/output audiovisual system and the keyboard or the touch screen (4):
• Select (110) the language of the application.
• Select (120) the functions from a group formed by: emotional inducements (121), mental trips (122), customized inducements (123) and download of new inducements (124).
• Select (180) the kind of the voice of the interaction and the complements like, for example, a male or female voice, rolling of the sea, aroma or ionization; and
• Implement (190) the selected function.
